# EUROPÄISCHE PATENTANMELDUNG

(11) **EP 0 687 465 A1**
(43) Veröffentlichungstag der Anmeldung: **20.12.1995**
(21) Anmeldenummer: 94107523.6
(22) Anmeldetag: 16.05.1994
(51) Int. Cl.: A61K 31/00, A61K 31/70, A61K 31/19

(54) **Verwendung von Glycyrrhizinsäure und ihrer Metabolite (Glycyrrhetinsäure) als Wirkstoff zur Herstellung eines Arzneimittels zur Therapie von viralen und allergischen Erkrankungen des Menschen**

(71) Anmelder: Albrecht, Uwe, Dipl.-Dok., D-30938 Burgwedel (DE); Schuhmaier, Adolf, D-69429 Waldbrunn (DE); AKUNA GESELLSCHAFT FÜR KLASSISCHE CHINESISCHE MEDIZIN mbH, D-69429 Waldbrunn 1 (DE)
(72) Erfinder: Albrecht, Uwe, Dipl.-Dok., D-30938 Burgwedel (DE); Schuhmaier, Adolf, D-69429 Waldbrunn (DE); AKUNA GESELLSCHAFT FÜR KLASSISCHE CHINESISCHE MEDIZIN mbH, D-69429 Waldbrunn 1 (DE)

(57) **Zusammenfassung**

Der Wirkstoff Glycirrhizinsäure wird innovativ zur Therapie viraler und allergischer Erkrankungen des Menschen eingesetzt. Der Wirkstoff kennzeichnet sich durch hervorragende Verträglichkeit und Wirksamkeit und stellt auf diesem Gebiet eine wichtige Neuerung der medizinischen Therapiemöglichkeiten mit Arzneimitteln dar. Die Darreichungsformen dieses Arzneimittels sollen vor allem zur Injektion, aber auch zur oralen Gabe bestimmt sein.

## Beschreibung

### Technisches Gebiet, auf das sich die Erfindung bezieht

Die Erfindung bezieht sich auf das Gebiet der Medizin, hier auf Glycirrhizinsäure und ihre Metabolite, die aus der Süßholzwurzel (Glycyrrhiza glabra L.) gewonnen wird. Dieser extrahierte Wirkstoff soll nun bei der Herstellung von Arzneimitteln in verschieden Darreichungsformen als Wirkstoff Einsatz finden in der Therapie verschiedener viraler und allergischer Erkrankungen des Menschen.

### Einschlägiger Stand der Technik

Glycirrhizinsäure wird aus den Wurzeln von Glcyrrhizia glabra gewonnen, wobei dieser Stoff und seine Derivate den isolierten Terpenverbindungen der Gruppe der pentozyklischen Triterpene zuzuordnen ist. Das reine getrocknete Pulver ist von weißer Farbe mit einem stark süßlichen Geschmack, einem Schmelzpunkt von ca. 220 Grad Celsius und als wäßrige Lösung stark schäumend. Die verschiedenen Inhaltsstoffe der Süßholzwurzel werden in unterschiedlichen Gebieten der Technik verwendet:
- Textilindustrie,
- Lederindustrie,
- Tabakindustrie,
- Metallurgie,
- Lebensmittelindustrie (z.B. Bier-, Likör-, Bonbonproduktion)
- chemische Industrie:
- Seifen- und Kosmetikproduktion,
- Zusatz zu Mundwassern, Zahnpasten,
- Arzneimittelherstellung
Der Bestandteil der Süßholzwurzel Glycirrhizinsäure wird derzeit in der Arzneimittelherstellung als Geschmackskorrigens, Hilfsstoff, oder Wirkstoff verwendet. Bekannt ist hier die Herstellung von Arzneimitteln zur Therapie von gastrointestinalen Erkrankungen und Urologika. Die Wirkungen der Glycirrhizinsäure in diesen Gebieten sind anhand der Literatur ausreichend belegt und zudem teilweise traditionell überliefert.

### Würdigung des Standes der Technik

In der Therapie der viralen und allergischen Erkrankungen des Menschen werden derzeit unterschiedliche Wirkstoffe eingesetzt. Insbesondere die antiviralen Wirkstoffe zeigen eine hohe Rate von unerwünschten Arzneimittelwirkungen und führen häufig nicht zu dem gewünschten Therapieerfolg. Hier sei beispielhaft die Gruppe der Interferone in der Therapie von viralen Lebererkrankungen und deren Folgezustände genannt.

### Darstellung der Erfindung

Zur Herstellung eines Arzneimittels zur Therapie der viralen und allergischen Erkrankungen des Menschen wird nun erfindungsgemäß Glycirrhizinsäure (bzw. deren Metabolite) als Wirkstoff eingesetzt. Hierbei wird vor allem eine injizierbare Zubereitung, die Glycirrhizin als aktiven Wirkstoff enthält, eingesetzt. Auch entsprechend hergestellte feste, zur oralen Einnahme bestimmte Arzneiformen mit dem Wirkstoff Glycirrhizin, kommen zum Einsatz.

### Vorteilhafte Wirkung der Erfindung

Die erfindungsgemäßen Arzneiformen können in einem weiten Indikationsbereich eingesetzt werden. Bei der Behandlung von viralen Lebererkrankungen und deren Folgezuständen stehen derzeit nur mit hohen unerwünschten Wirkungen behaftete Arzneimittel zur Verfügung. Die erfindungsgemäßen Zubereitungen mit Glycirrhizinsäure haben bei hohem therapeutischen Wert eine geringe Rate von unerwünschten Arzneimittelwirkungen. Darüberhinaus sind derzeit keine den hohen ethischen Ansprüchen bei der Behandlung komplizierter viraler Erkrankungen gerecht werdende Wirkstoffe verfügbar. Gerade in diesem Bereich stellt der Einsatz des Wirkstoffes Glycirrhizin zur Herstellung eines Arzneimittels einen bedeutenden Fortschritt der therapeutischen Möglichkeiten der Medizin dar.

### Beschreibung eines Weges zur Ausführung der Erfindung

Bei der Zubereitung der Injektionslösung liegt zunächst Glycirrhizin als weißes, kristallines Pulver mit stark süßlichem Geschmack vor. Dieses ist in kaltem Wasser nahezu unlöslich, wohl aber frei löslich in erwärmten, verdünnten Ethanol, erwärmter Essigsäure oder Ammoniumlösung.

Generika-Name: Glycirrhizin Chemischer Name: 20 β-carboxy-11-oxo-30-norolean-12-en-3 β-yl-2-O-β-D-glucopyranuronosyl-β-D - glucopyranosiduronic- Säure Molekularformel: C 42 - H 62 - O 16 Molekulargewicht: 822.92 Schmelzpunkt: 214 - 228 Grad Celsius

Es wird eine farblose, transparente, wässrige Lösung als injizierbare Lösung hergestellt. Der pH-Wert liegt zwischen 6.0 und 7.4. Die "Osmotic Pressure Ratio" bezogen auf physiologische Kochsalzlösung beträgt ca. 2.

Die fertige Lösung wird unter strikter Beachtung der "Good Manufacturing Practice" analysiert. Es werden dann additionale Wirkstoffe (z.B. Essigsäure und L-Cystein-Hydrochlorid) und Hilfsstoffe zugesetzt. Danach erfolgt unter sterilen Bedingungen die Abfüllung in Ampullen. Die so fertiggestellte Arzneiform ist zur intravenösen Verabreichung bestimmt.

## Patentansprüche

1. Virale Erkrankungen: Behandlung verschiedender Formen der viralen Erkrankungen der Leber und deren Folgezustände, Herpes-Simplex-Virus-Erkrankungen, Varicella-Zoster-Erkrankungen, Erkrankungen infolge einer HIV-Virus-Infektion.

2. Allergische Erkrankungen: Strophulus infantum, Ekzema dermatitis, Urticaria, Pruritus, Arzneimittelekzem, Stomatitis.
